# EUROPEAN PATENT SPECIFICATION

(11) **EP 4 510 936 B1**
(45) Date of publication and mention of the grant of the patent: **21.10.2026**
(21) Application number: 23724970.1
(22) Date of filing: 18.04.2023
(51) Int. Cl.: A61B 6/04

(54) **VARIABLE ANGLE PATIENT SUPPORT SYSTEM FOR RADIATION TREATMENT**
WINKELVERSTELLBARES PATIENTENSTÜTZSYSTEM ZUR STRAHLENBEHANDLUNG
SYSTÈME DE SUPPORT DE PATIENT À ANGLE VARIABLE POUR RADIOTHÉRAPIE

(30) Priority: 21.04.2022 US 202263333342 P; 04.05.2022 US 202263338120 P
(43) Date of publication of application: 26.02.2025
(73) Proprietor: MEDTEC LLC, Orange City, IA 51041 (US)
(72) Inventor: WILSON, Roger F., Sarasota, Florida 34240 (US); DURAND, Renaud, Iowa City, Iowa 52246 (US); DOSTER, Tauni, Iowa City, Iowa 52245 (US); SCHWAB, Benjamin, Eureka, Missouri 63025 (US)
(74) Representative: Zacco Denmark A/S
(86) International application number: PCT/US2023/018972
(87) International publication number: WO 2023/205167

(56) References cited:
- CN-A- 112 891 120
- CN-B- 109 602 569
- US-A- 3 446 532
- US-A- 5 316 370
- US-A1- 2011 143 898
- US-A1- 2014 325 759
- US-B1- 6 698 045

## Description

### BACKGROUND

Certain types of medical treatments or tests require that a portion of a human body be held in a same position to facilitate performance of the medical treatment or test upon that portion of the body. For example, when breast cancer patients undergo radiation treatment, their upper bodies and breast(s) must be maintained in a precise, repeatable location for the treatment such that the underlying position of the breast tumor is fixed in space for the duration of the radiation treatment. Various different techniques have been used in the field of radiation oncology for supporting and holding body parts in a fixed position. In this context, a technical teaching provided in US 5 316 370 A is acknowledged.

### BRIEF DESCRIPTION OF THE DRAWINGS

The invention is defined in the appended set of claims. Details of the system provided by the invention are explained hereinafter.
FIG. 1 illustrates an exemplary implementation of a patient support system described herein;
FIG. 2 illustrates an example of a lok bar and track for positioning a body part support module upon a tiltable support board of the patient support system;
FIGs. 3A and 3B illustrate examples of a body support module that may be used with the patient support system;
FIGs. 4A-4C illustrate an example of an angle adjustment mechanism that supports the tiltable patient support board and which enables an adjustment of a tilt angle of the patient support board;
FIG. 5 depicts an example of adjustment of the tilt angle of the tiltable support board of the patient support system;
FIGs. 6A-6D illustrate the tiltable support board adjusted to various specific tilt angles;
FIGs. 7-11B illustrate a first implementation of a patient bottom support module; and
FIGs. 12-13 illustrate a second implementation of the patient bottom support module.

### DETAILED DESCRIPTION OF THE PREFERRED EMBODIMENTS

The following detailed description refers to the accompanying drawings. The same reference numbers in different drawings may identify the same or similar elements. The following detailed description does not limit the invention.

A wide variety of existing systems for supine cancer treatment are available for sale across the world. These existing systems rest on support couches and provide means for positioning a supine patient at various angles of inclination for external beam radiation treatment of cancer in the thorax, breast, and abdomen. Some are, for example, operable within Magnetic Resonance Imaging (MRI) environments. The range and complexity of adjustment for patient and device positioning of these existing systems varies, as does effectiveness, efficiency, and cost. High levels of adaptability in arm supports, or angle adjustment, of existing systems can be advantageous to patient comfort and treatment needs, but the associated complexity too often strains the clinician's mechanical skills and takes time busy clinics do not have. Reliable device and patient positioning is crucial, but given the above-noted short comings of the existing patient support systems, clinics, doctor's offices, and hospitals often resort to a generic set up, for simplicity and speed, which often is not optimal for patient comfort or treatment. Even in cases where the means to adjust the angle settings, for instance, is straight forward, excess complexity is usually still evident in existing systems, with the inclusion of numerous parts that add weight and cost without necessarily ensuring against other drawbacks or limitations. Additionally, present products/systems on the market offer few configuration options, thus, limiting a clinic's or a hospital's ability to tailor the system to best fit their needs and budget.

A modular patient support system described herein provides flexibility in choosing the overall configuration that best suits the needs of the patient and the needs to achieve the desired treatment. The modular patient support system has an effective minimalist approach, while maintaining the crucial elements required for effective treatment. Simple, quick, and accurate set-up of the modular patient support system, over a wide range of patient sizes, is accomplished without compromising comfort or treatment quality. The modular patient support system has adjustable angles of inclination for immobilizing portions of the body of a patient for treatment, testing, or examination. In particular, implementations described herein may provide patient support during testing or treatment of the breast, thorax, or other upper body parts.

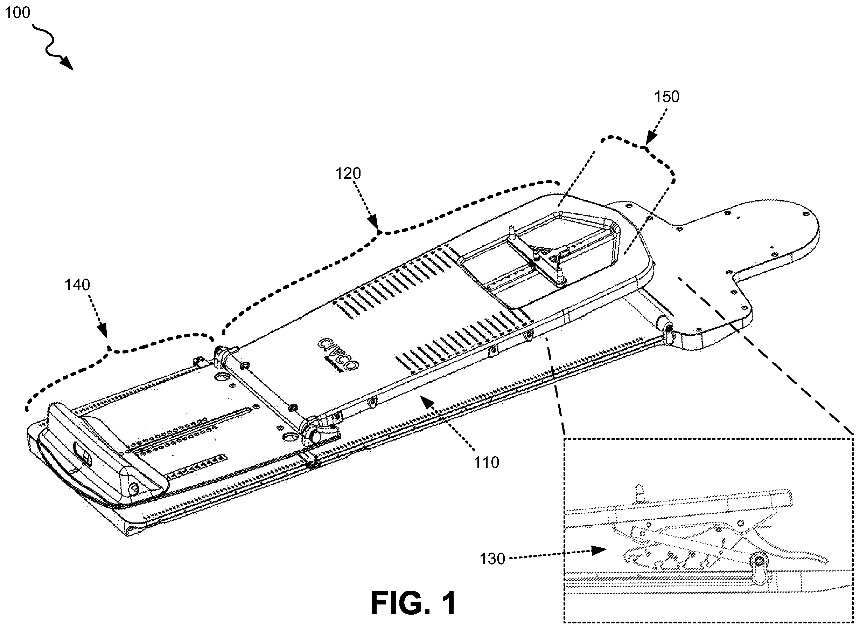

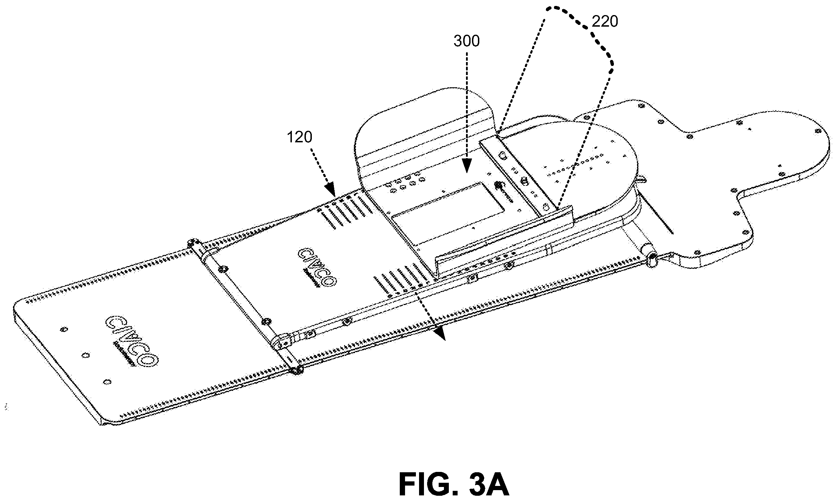

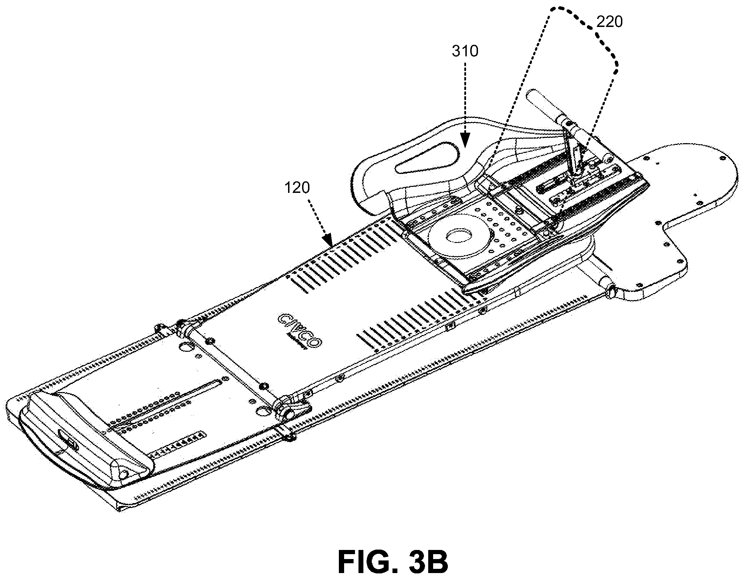

As shown in FIG. 1, the modular patient support system 100 includes a baseboard 110, a tiltable support board 120, an angle adjustment mechanism 130, and a patient bottom support module 140. The baseboard 110 may include a planar, generally rectangular board upon which the tiltable support board 120 rests and may be an upper surface of a support table or support couch. The angle adjustment mechanism 130 may include a structure, described further below, that extends between an underside of the tiltable patient support board 120 and an upper surface of the baseboard 110 to support that patient support board 120 upon the baseboard 110. The bottom support module 140 may be removably attachable to hinging points upon an inferior, pivoting end of the tiltable support board 120. Various different body part support modules may be attached to a mounting region 150 on an upper surface of the tiltable support board 120 to provide support for specific body parts of the patient (e.g., head, arms). Examples of body part support modules are shown in FIGs. 3A and 3B.

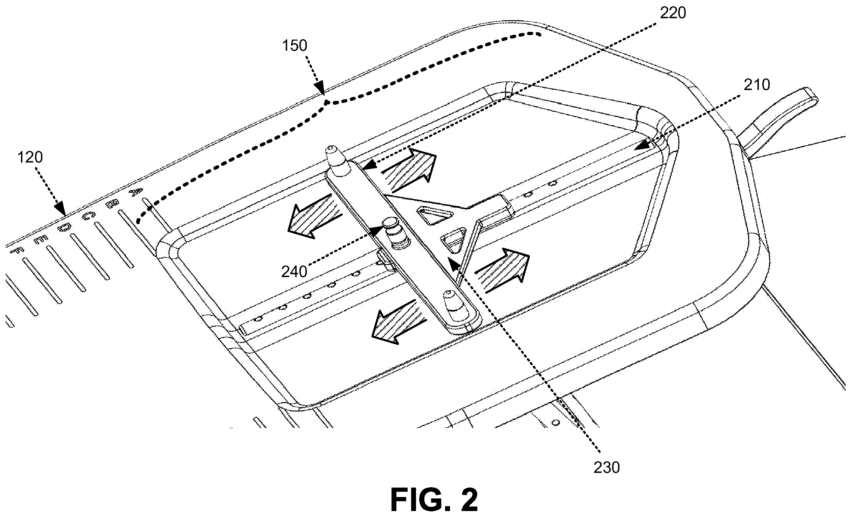

As further shown in FIG. 2, the tiltable support board 120 includes, at a first or superior end of the support board 120, a body support module mounting region 150. The body support module mounting region 150 includes a recessed area in which a track 210 is disposed longitudinally. A universal style lok bar 220 (e.g., a three pin lok bar) mounts to a frame 230, which further mounts on the track 210. Frame 230, along with lok bar 220, can be slid along the track 210 and repositioned at multiple registered positions on the track 210. A releasable spring plunger 240 permits incremental adjustment of the lok bar 220 along the track 210 at the multiple registered positions. When the body support module (not shown) is attached to the lok bar 220, the lok bar 220 can be repositioned at one of the multiple positions along the track 210 such that the attached body support module can be moved up or down the upper surface of the tiltable support board 120. In one implementation, such as shown in FIG. 2, the releasable spring plunger 240 may be located at a center pin location upon lok bar 220. A close tolerance fit between the lok bar 220, frame 230, and the track 210 minimizes yaw play. The universal lok bar pin registration allows for use of several different existing body part support modules (e.g., wingboards) that are configured to attach to the universal lok bar 220. Lok bar 220 may include, for example, a two or three pin lok bar.

FIGs. 3A and 3B depict two examples of different designs of body support modules 300 and 310 that may be attached to lok bar 220 upon tiltable support board 120. Body support modules 300 and 310, shown in FIGs. 3A and 3B, are examples of "wingboards" that support a patient's head, upper back, shoulders, and the patient's arms in an "arms-raised-over-the-head" position upon the underlying tiltable support board 120. Other body support modules, different than those shown in FIGs. 3A and 3B, may have designs that enable attachment to lok bar 220 on the upper surface of tiltable support board 120.

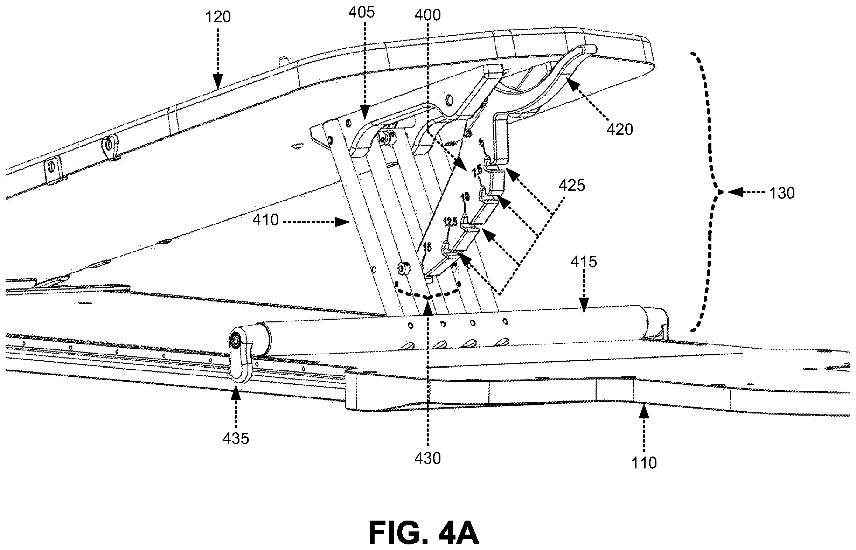

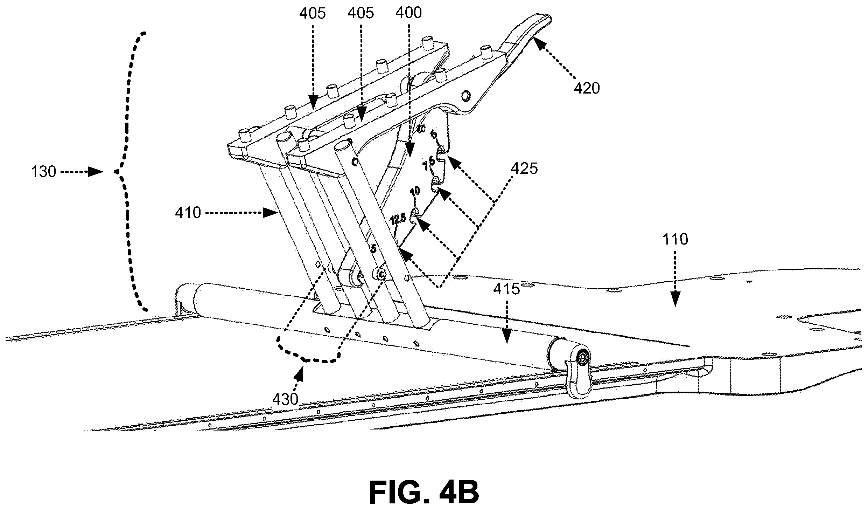

FIGs. 4A-4C illustrate an example of angle adjustment mechanism 130 that supports tiltable patient support board 120 upon baseboard 110 and which enables an adjustment of a tilt angle between patient support board 120 and baseboard 110. As shown in FIGs. 4A and 4B, the angle adjustment mechanism 130 includes a structure that provides multiple tilt angle settings (e.g., 5, 7.5, 10, 12.5 and 15 degrees) between the underlying baseboard 110 and the tiltable support board 120. The "scissors-like" structure of the angle adjustment mechanism 130 includes multiple interworking elements that are designed to hold the support board 120 in a specific one of the adjustment angle settings, but also prevent inadvertent release of the patient support board 120. The angle adjustment mechanism 130 includes an adjustment bracket 400, two mounting brackets 405, multiple legs 410, and a support foot 415.

Adjustment bracket 400 may have an arcuate shape and may include an integral adjustment handle 420 at one end of the bracket 400, and multiple retaining notches 425 disposed along an underside of the bracket 400. One of the retaining notches 410 may be engaged, at a second, opposite end of the bracket 400, with a cross-bar 430 that extends between, and connects, at least two of the legs 410. Cross-bar 430 may extend transversely to legs 410. Adjustment bracket 400, when a retaining notch 425 is engaged with cross-bar 430, may form an acute angle with legs 410

Mounting brackets 405 attach to an underside of tiltable patient support board 120 and provide attachment points for attaching legs 410 so as to couple legs 410 to support board 120. Legs 410 may include multiple legs (e.g., four legs are shown in FIGs. 4A-4C) that attach to mounting brackets 405 at a first or superior end of legs 410 and attach to support foot 415 at a second or inferior end of legs 410. Legs 410 may extend parallel to, and equally spaced apart from, one another from an underside of patient support board 120 to support foot 415.

Support foot 415 may include a tubular member that may transversely attach to legs 410 and which may rest upon baseboard 110, transverse to a length of the baseboard 110, and provide a support base to support patient support board 120 upon baseboard 110. Support foot 415 may include pivotable tabs 435 at each end of the tubular member that capture sides of the baseboard 110 to align the tiltable support board 120 with the underlying baseboard and to minimize yaw and provide repeatable, accurate alignment of the tiltable support board 120 with the baseboard 110.

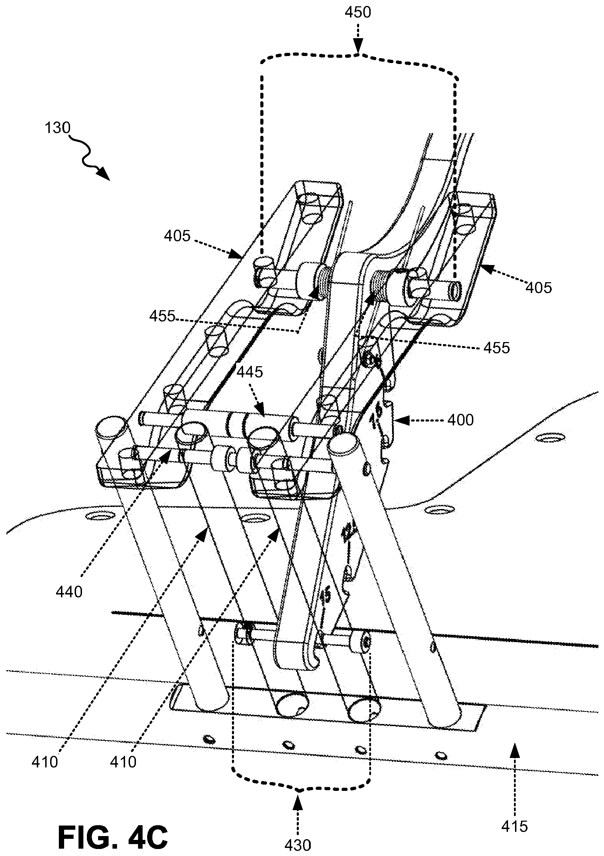

FIG. 4C depicts an isometric view of angle adjustment mechanism 130 with support brackets 405 and two interior legs 410 shown as transparent so that the interconnection of the various other components of the structure of angle adjustment mechanism 130 can be seen. As shown, a leg cross-bar 440 extends between, and through, a superior end of each of the legs 410 to operate as a pivot point upon which an inferior end of each of support brackets 405 pivot when the tilt angle of patient support board 120 (not shown) is being adjusted. A cross-bar 445 may further extend between each of support brackets 405 to maintain a spacing between the inferior ends of brackets 405 and to enhance the mechanical strength of the overall structure of angle adjustment mechanism 130. An adjustment bracket cross-bar 450 may also extend between each of support brackets 405 at a superior end of brackets 405 to provide a rotation axis about which adjustment bracket 400 may rotate when adjusting the tilt angle of patient support board 120 (not shown). A hole, having a diameter slightly larger than an exterior diameter of adjustment bracket cross-bar 450, may be disposed at a superior end of adjustment bracket 400 and through which cross-bar 450 may be inserted to position adjustment bracket 400 between support brackets 405 beneath patient support board 120 (not shown). Torsion springs 455 may be positioned, upon adjustment bracket cross-bar 450, at each side of adjustment bracket 400, with a superior arm of torsion springs 455 contacting an underside of the patient support board 120, and an inferior arm contacting a retention pin mounted to side surfaces of adjustment bracket 400, to provide a rotational bias force that holds an inferior end (i.e., a retention notch 425) of adjustment bracket 400 against cross-bar 430.

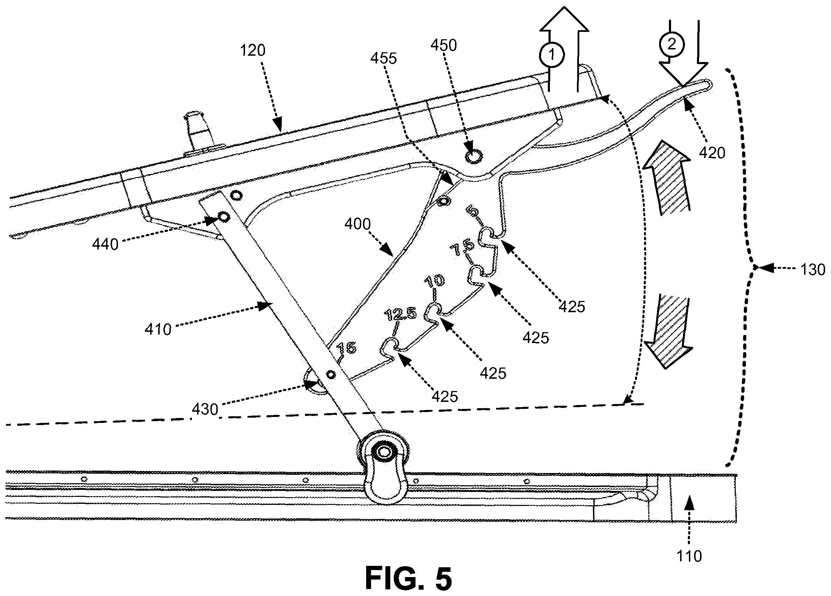

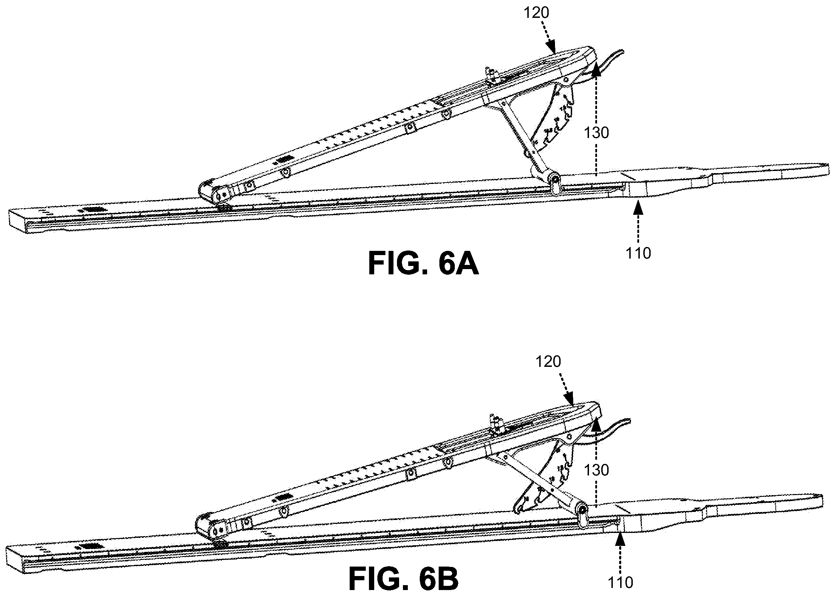

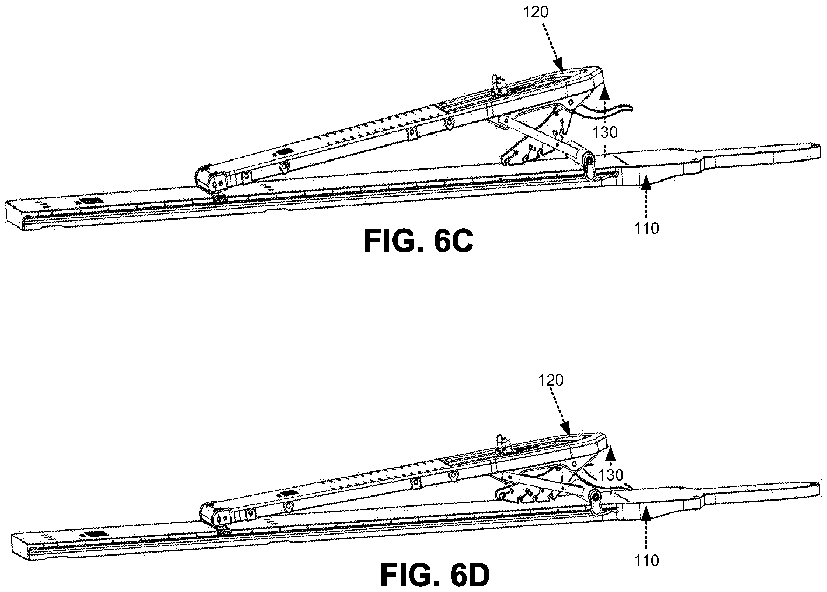

Referring to FIG. 5, to change an angle setting of the angle adjustment mechanism 130, an operator must first raise the superior end of the support board 120 and simultaneously press downward upon the adjustment handle 420 of adjustment bracket 400 to disengage the current retaining notch 425 that is engaged with cross-bar 430. The operator may then adjust the angle setting of the tiltable support board 120 to select a different retaining notch 425 (e.g., one of 5, 7.5, 10, 12.5 or 15 degrees) upon the bracket 400, and lower the superior end of the tiltable support board 120 to cause the new retaining notch 425 to engage with cross-bar 430 at the desired tilt angle of the tiltable support board 120. This configuration of the angle adjustment mechanism 130 maintains the position of the support board 120 relative to the baseboard 110 even when, for example, the entire patient support system 100 is moved. The minimization of internal forces in the design of the angle adjustment mechanism 130, shown in FIGs. 4A - 4C and 5, reduces cost and helps to ensure greater reliability and safety. FIG. 6A illustrates an example of angle adjustment mechanism 130 adjusted to a maximum tilt angle of 15 degrees, and FIG. 6B shows an example of angle adjustment mechanism 130 adjusted to a tilt angle of 10 degrees. FIG. 6C illustrates an example of angle adjustment mechanism 130 adjusted to a tilt angle of 7.5 degrees, and FIG. 6D shows an example of angle adjustment mechanism 130 adjusted to a tilt angle of 5 degrees.

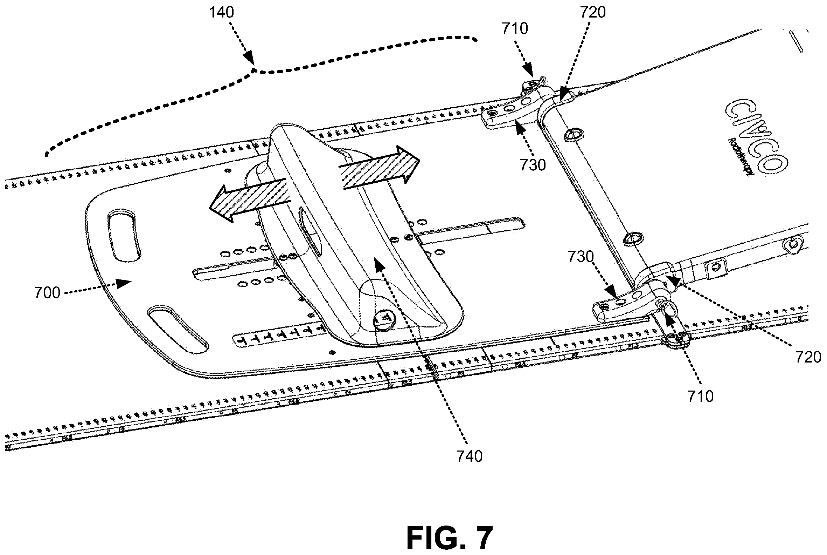

FIGs. 7-11B depict a first implementation of patient bottom support module 140. As shown in FIG. 7, the patient bottom support module 140 includes a planar bottom support board 700 that attaches or detaches from a first or inferior end of the tiltable support board 120 via releasable spring plungers 710 that connect a hinging pivot point 720 at the inferior end of the tiltable support board 120 with hinges 730 upon the bottom support board 700. The bottom support board 700 further includes a slidable raised bottom stop 740 that includes an underlying ratchet-like means (not shown in FIG. 7) to slide, register, and secure the raised bottom stop upon spaced holes located at various longitudinal positions along the bottom support board 700. At an extreme inferior end of travel, the slidable raised bottom stop 740 and the underlying ratchet may together be extended further away from tiltable support board 120 then what is provided for by the registered positions to allow more room for the patient to climb on and off the system. To reduce torque and strain on the attachment points to the tiltable support board 120 when a patient is climbing off and on, a number of rubber feet (not shown) may be disposed to protrude below the bottom support board 700 to rest against the baseboard 110.

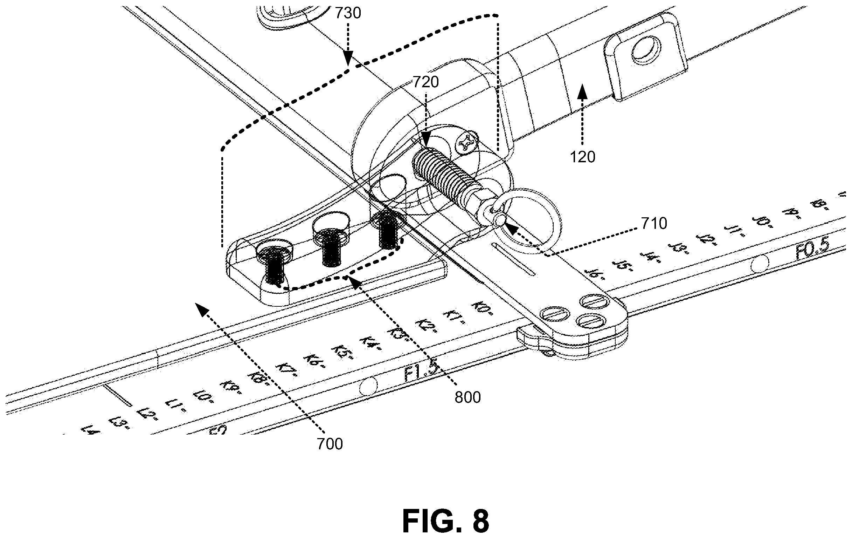

FIG. 8 illustrates a close-up isometric view of the attachment between bottom support board 700 and tiltable patient support board 120, with hinge 730 being shown as transparent. As shown, hinge 730 attaches to bottom support board 700 via, for example, multiple screws 800 that insert through hinge 730 into bottom support board 700. Hinge 730 includes, at an end opposite of the screw attachment to bottom support board 700, a hole that aligns with a counterpart hole in the hinging pivot point 720 of patient support board 120. A releasable spring plunger 710 inserts into the hole in hinge 730 to screw into the counterpart hole in the hinging pivot point 720. Releasable spring plunger 710 may be pulled in an outwards direction to cause the spring plunger 710 to release its connection with the counterpart hole in the hinging pivot point 720 such that bottom support module 140 may be removed from its attachment to patient support board 120.

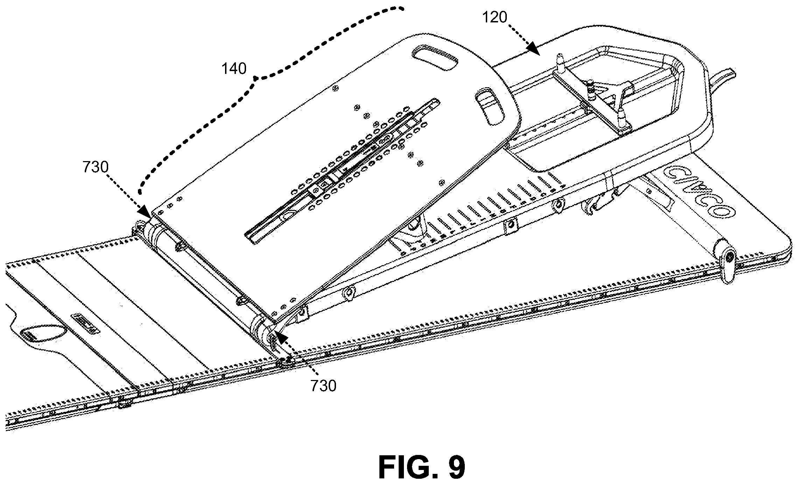

FIG. 9 depicts bottom support module 140 in a "folded over" position in which bottom support module 140, via hinges 730 at the inferior end of tiltable patient support board 120, is rotated such that a superior side of bottom support module 140 rests upon the upper or superior surface of patient support board 120. Folding bottom support module 140 to rest upon the upper surface of tiltable support board 120 creates a more compact system for easier transport.

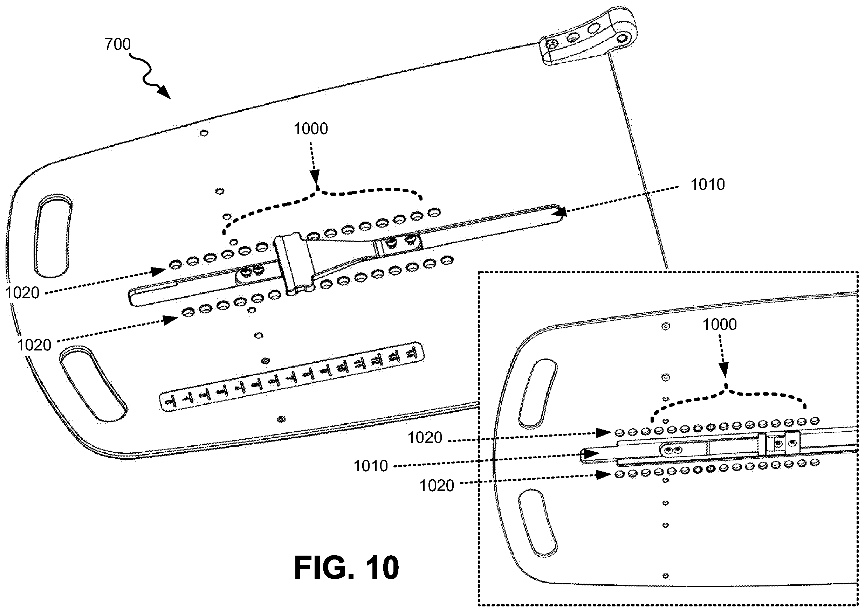

FIG. 10 depicts the ratchet mechanism 1000 disposed in bottom support board 700. A longitudinal slot 1010 extends across a midline of bottom support board 700 and operates as a track in which the ratchet mechanism 1000 slides longitudinally to adjust a position of the raised bottom stop 740 (not shown) that is attached to ratchet mechanism 1000. The ratchet mechanism 1000 engages with a linear series of spaced holes in bottom support board 700, as described further below.

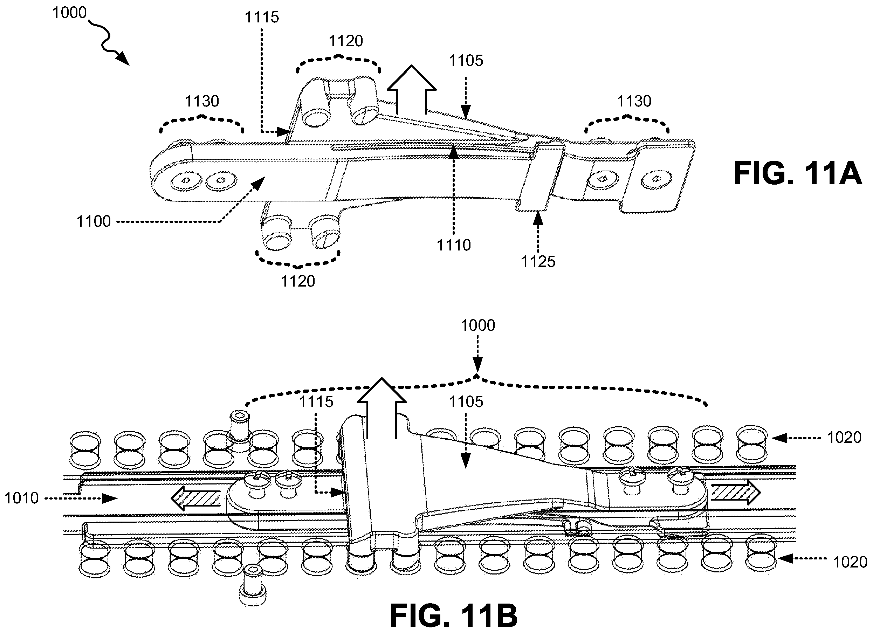

FIG. 11A illustrates design details of an example of ratchet mechanism 1000. Ratchet mechanism 1000 may be formed from a flexible material, such as, for example, a plastic material. Ratchet mechanism 1000 includes an integrally formed member having a first portion 1100 connected to a second portion 1105 via a thin, flexible portion 1110. Second portion 1105 includes a ratchet portion 1115 that further includes retention pins 1120 that are configured to extend into the linear series of spaced holes in bottom support board 700 (not shown). First portion 1100 includes a retaining member 1125 that extends below a lower surface of bottom support board 700 and holds ratchet mechanism 1000 within longitudinal slot 1010. As further shown, ratchet mechanism 1000 includes connection screws 1130 disposed at each end of mechanism 1000 for connecting mechanism 1000 to the raised bottom stop 740 (not shown).

FIG. 11B depicts an example of the use of ratchet mechanism 1000 within longitudinal slot 1010 of bottom support board 700. To change a registered position of raised bottom stop 740, an operator (not shown) raises the flexible second portion 1105 away from the lower first portion 1100 to extract or release the retention pins 1120 from the spaced holes 1020 in bottom support board 700, and then applies force to the raised bottom stop 740 to push the raised bottom stop 740 in either longitudinal direction upon bottom support board 700 (e.g., either left or right, in the view of FIG. 11B). Adjustment of the raised bottom stop 740 upon bottom support board 700 enables the raised bottom stop 740 to be positioned for use by different people having different sizes and to hold their bodies at a particular position upon patient support board 120 without having the people slip downwards upon bottom support board 700.

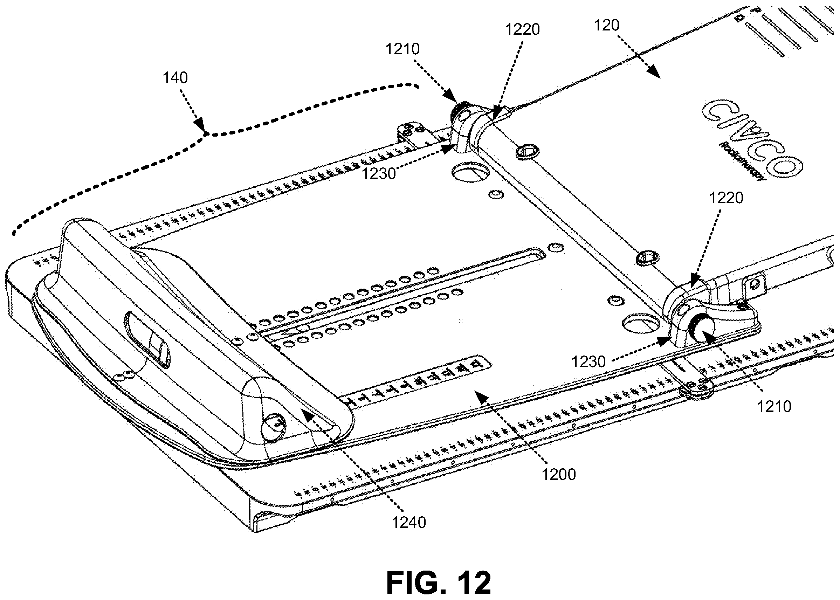

FIGs. 12-13 depict a second implementation of patient bottom support module 140. As shown in FIG. 12, the patient bottom support module 140 includes a planar bottom support board 1200 that attaches or detaches from an inferior end of the tiltable support board 120 via threaded bolts 1210 that connect hinging pivot points 1220 at the inferior end of the tiltable support board 120 with hinges 1230 upon the bottom support board 1200. The bottom support board 1200 further includes a slidable raised bottom stop 1240 that includes an underlying ratchet-like means (not shown in FIG. 12) to slide, register, and secure the raised bottom stop upon spaced holes located at various longitudinal positions along the bottom support board 1200. Unlike the first implementation shown in FIG. 7, bottom support board 1200 extends under an inferior end of patient support board 120, and the hinges 1230 are reversed in orientation relative to the first implementation shown in FIG. 7.

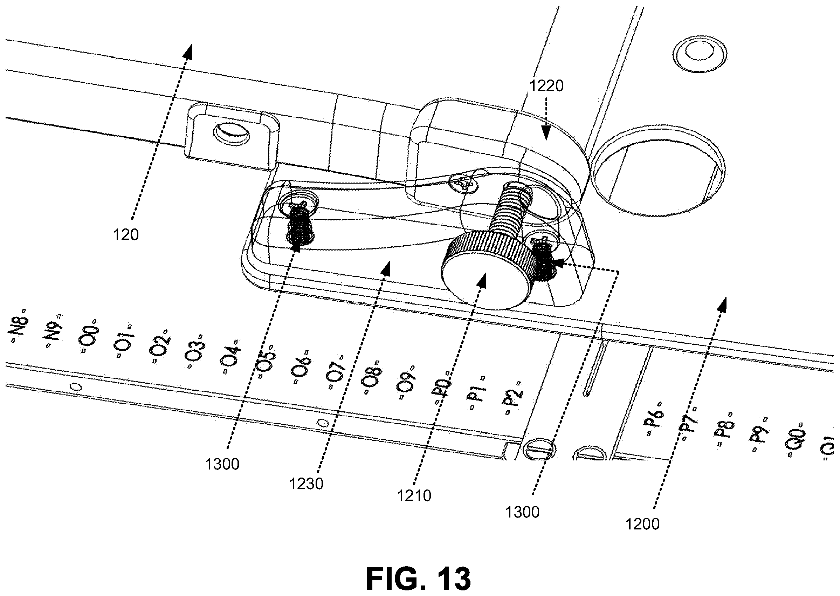

FIG. 13 illustrates a close-up isometric view of the attachment between bottom support board 1200 and tiltable patient support board 120, with hinge 1230 being shown as transparent. As shown, hinge 1230 attaches to bottom support board 1200 via, for example, multiple screws 1300 that insert through hinge 1230 into bottom support board 1200. Hinge 1230 includes, at one end, a hole that aligns with a counterpart hole in the hinging pivot point 1220 of patient support board 120. A threaded bolt 1210 threads into the hole in hinge 1230 to screw into the counterpart hole in the hinging pivot point 1220. Threaded bolt 1210 may be unscrewed from the hole in hinge 1230 to release the connection with the counterpart hole in the hinging pivot point 1220 such that bottom support module 140 may be removed from its attachment to patient support board 120.

The foregoing description of implementations provides illustration and description, but is not intended to be exhaustive or to limit the invention to the precise form disclosed. Modifications and variations are possible in light of the above teachings or may be acquired from practice of the invention.

Although the invention has been described in detail above, it is expressly understood that it will be apparent to persons skilled in the relevant art that the invention may be modified without departing from the scope of the invention. Various changes of form, design, or arrangement may be made to the invention without departing from the scope of the invention. Therefore, the above-mentioned description is to be considered exemplary, rather than limiting, and the true scope of the invention is that defined in the following claims.

No element, act, or instruction used in the description of the present application should be construed as critical or essential to the invention unless explicitly described as such. Also, as used herein, the article "a" is intended to include one or more items. Further, the phrase "based on" is intended to mean "based, at least in part, on" unless explicitly stated otherwise.

## Claims

1. A system for positioning a patient, comprising:
a tiltable patient support board (120); and
an angle adjustment mechanism (130) mounted to a lower surface of the tiltable patient support board and configured to adjust a tilt angle of the tiltable patient support board, wherein the angle adjustment mechanism comprises:
a plurality of legs (410) coupled to an underside of the tiltable patient support board,
a cross-bar (430) extending between at least two of the plurality of legs, and
a bracket (400) coupled to the underside of the tiltable patient support board at a first end of the bracket, and coupled to the plurality of legs, wherein the bracket and the plurality of legs form an angle with one another, wherein the bracket comprises a handle (420) and multiple retaining notches (425),
and wherein the bracket is configured to be adjusted, via use of the handle, to selectively engage one of the multiple retaining notches with the cross-bar to adjust the tilt angle of the tiltable patient support board.

2. The system of claim 1, wherein the multiple retaining notches are disposed along an underside of the bracket, and wherein the underside of the bracket couples to the at least two of the plurality of legs via engagement of the one of the multiple retaining notches with the cross-bar.

3. The system of claim 1, further comprising:
a tubular foot (415), wherein the plurality of legs connects in parallel between the underside of the tiltable patient support board and the tubular foot and are spaced apart from one another.

4. The system of claim 1, wherein the multiple retaining notches are disposed, and spaced apart, along an underside of the bracket and wherein selective engagement of each different one of the multiple retaining notches with the cross-bar changes the tilt angle of the tiltable patient support board to a different one of multiple tilt angles.

5. The system of claim 1, wherein the bracket comprises an arcuate-shaped bracket, wherein the handle comprises an integral adjustment handle, and wherein the multiple retaining notches are disposed along an underside of the bracket to enable changing a tilt angle setting of the angle adjustment mechanism to adjust the tilt angle of the patient support board.

6. The system of claim 1, further comprising: a foot (415) connected transversely to the plurality of legs and configured to rest upon an underlying support surface.

7. The system of claim 6, further comprising: pivotable tabs (435) connected to each end of the foot and configured to capture sides of the underlying support surface.

8. The system of claim 1, further comprising:
a detachable patient bottom support board that removably attaches to the tiltable patient support board at a hinged attachment point.

9. The system of claim 8, wherein the patient bottom support board removably attaches to the tiltable patient support board via at least one of releasable spring plungers or threaded bolts.

10. The system of claim 1, wherein the tiltable patient support board includes a lok bar that slidably engages with a track within a recess of the tiltable patient support board and wherein a patient body part support module removably attaches to the lok bar.

11. The system of claim 1, wherein the tiltable patient support board further comprises:
a recess disposed within an upper surface of the patient support board,
a slidable track affixed within the recess,
a frame configured to slide upon the slidable track, and
a lok bar attached to the frame and configured to permit attachment and detachment of a body support module to the lok bar, and further having a releasable spring plunger for repositioning the body support module at a plurality of positions along the slidable track.

12. The system of claim 1, wherein the tiltable patient support board supports a portion of an upper body of a patient.

13. The system of claim 1, further comprising:
a detachable bottom support module that attaches to the tiltable patient support board at hinged attachment points, wherein the bottom support board module further comprises a bottom support board that supports a portion of a lower body of the patient and that is rotatable, via the hinged attachment points, to cause the bottom support board to rotate towards a superior end of the tiltable patient support board and rest upon an upper surface of the tiltable patient support board.

14. The system of claim 13, wherein the bottom support module further comprises a raised bottom stop that slidably attaches to the bottom support board.

15. The system of claim 14, wherein the bottom support module further comprises a ratchet mechanism that enables the raised bottom stop to slide upon the bottom support board to one of multiple registered positions on the bottom support board.

## Patentansprüche

1. System zur Positionierung eines Patienten, umfassend:
eine neigbare Patientenauflageplatte (120); und
einen Winkelverstellmechanismus (130), der an einer Unterseite der neigbaren Patientenauflageplatte montiert und dazu konfiguriert ist, einen Neigungswinkel der neigbaren Patientenauflageplatte einzustellen, wobei der Winkelverstellmechanismus umfasst:
eine Mehrzahl von Beinen (410), die mit einer Unterseite der neigbaren Patientenauflageplatte gekoppelt sind, eine Querstrebe (430), die sich zwischen mindestens zwei der Mehrzahl von Beinen erstreckt, und einen Halter (400), der an einem ersten Ende des Halters mit der Unterseite der neigbaren Patientenauflageplatte gekoppelt ist und mit der Mehrzahl von Beinen gekoppelt ist, wobei der Halter und die Mehrzahl von Beinen einen Winkel zueinander bilden, wobei der Halter einen Griff (420) und mehrere Rastkerben (425) umfasst, und wobei der Halter dazu konfiguriert ist, mittels Verwendung des Griffs verstellt zu werden, um selektiv eine der mehreren Rastkerben mit der Querstrebe in Eingriff zu bringen, um den Neigungswinkel der neigbaren Patientenauflageplatte einzustellen.

2. System nach Anspruch 1, wobei die mehreren Rastkerben entlang einer Unterseite des Halters angeordnet sind, und wobei die Unterseite des Halters mit den mindestens zwei der Mehrzahl von Beinen durch Eingriff der einen der mehreren Rastkerben mit der Querstrebe gekoppelt wird.

3. System nach Anspruch 1, ferner umfassend:
einen rohrförmigen Fuß (415),
wobei die Mehrzahl von Beinen parallel zwischen der Unterseite der neigbaren Patientenauflageplatte und dem rohrförmigen Fuß verbunden und voneinander beabstandet ist.

4. System nach Anspruch 1, wobei die mehreren Rastkerben entlang einer Unterseite des Halters angeordnet und voneinander beabstandet sind, und wobei der selektive Eingriff jeder unterschiedlichen der mehreren Rastkerben mit der Querstrebe den Neigungswinkel der neigbaren Patientenauflageplatte auf jeweils einen unterschiedlichen von mehreren Neigungswinkeln verändert.

5. System nach Anspruch 1, wobei der Halter einen bogenförmigen Halter umfasst, wobei der Griff einen integralen Verstellgriff umfasst, und wobei die mehreren Rastkerben entlang einer Unterseite des Halters angeordnet sind, um eine Änderung einer Neigungswinkeleinstellung des Winkelverstellmechanismus zu ermöglichen, sodass der Neigungswinkel der Patientenauflageplatte eingestellt wird.

6. System nach Anspruch 1, ferner umfassend:
einen Fuß (415), der quer zur Mehrzahl von Beinen verbunden und dazu konfiguriert ist, auf einer darunterliegenden Trägerfläche aufzuliegen.

7. System nach Anspruch 6, ferner umfassend:
schwenkbare Laschen (435), die mit jedem Ende des Fußes verbunden und dazu konfiguriert sind, Seiten der darunterliegenden Trägerfläche zu erfassen.

8. System nach Anspruch 1, ferner umfassend:
eine abnehmbare untere Patientenauflageplatte, die an einem gelenkigen Befestigungspunkt lösbar an der neigbaren Patientenauflageplatte befestigt wird.

9. System nach Anspruch 8, wobei die untere Patientenauflageplatte lösbar an der neigbaren Patientenauflageplatte mittels mindestens eines von lösbaren Federdruckbolzen oder Gewindebolzen befestigt wird.

10. System nach Anspruch 1, wobei die neigbare Patientenauflageplatte eine Indexierungsleiste umfasst, die gleitend mit einer Führung innerhalb einer Aussparung der neigbaren Patientenauflageplatte in Eingriff steht, und wobei ein Stützmodul für ein Körperteil eines Patienten lösbar an der Indexierungsleiste befestigt wird.

11. System nach Anspruch 1, wobei die neigbare Patientenauflageplatte ferner umfasst:
eine Aussparung, die innerhalb einer Oberseite der Patientenauflageplatte angeordnet ist,
eine verschiebbare Führung, die innerhalb der Aussparung befestigt ist,
einen Rahmen, der dazu konfiguriert ist, auf der verschiebbaren Führung zu gleiten, und
eine Indexierungsleiste, die an dem Rahmen befestigt und dazu konfiguriert ist, das Befestigen und Lösen eines Körperstützmoduls an der Indexierungsleiste zu ermöglichen, und die ferner einen lösbaren Federdruckbolzen zum Neupositionieren des Körperstützmoduls an einer Mehrzahl von Positionen entlang der verschiebbaren Führung aufweist.

12. System nach Anspruch 1, wobei die neigbare Patientenauflageplatte einen Teil eines Oberkörpers eines Patienten stützt.

13. System nach Anspruch 1, ferner umfassend:
ein abnehmbares unteres Stützmodul, das an gelenkigen Befestigungspunkten an der neigbaren Patientenauflageplatte befestigt wird, wobei das untere Stützmodul ferner eine untere Stützplatte umfasst, die einen Teil eines Unterkörpers des Patienten stützt und die mittels der gelenkigen Befestigungspunkte drehbar ist, um zu bewirken, dass sich die untere Stützplatte zu einem oberen Ende der neigbaren Patientenauflageplatte hin dreht und auf einer Oberseite der neigbaren Patientenauflageplatte aufliegt.

14. System nach Anspruch 13, wobei das untere Stützmodul ferner einen erhöhten unteren Anschlag umfasst, der gleitbar an der unteren Stützplatte befestigt wird.

15. System nach Anspruch 14, wobei das untere Stützmodul ferner einen Ratschenmechanismus umfasst, der es dem erhöhten unteren Anschlag ermöglicht, auf der unteren Stützplatte zu einer von mehreren vorgegebenen Positionen auf der unteren Stützplatte zu gleiten.

## Revendications

1. Système pour positionner un patient, comprenant :
une plaque de support de patient inclinable (120) ; et
un mécanisme de réglage d'angle (130) monté sur une surface inférieure de la plaque de support de patient inclinable et configuré pour régler un angle d'inclinaison de la plaque de support de patient inclinable, dans lequel le mécanisme de réglage d'angle comprend :
une pluralité de montants (410) couplés à une face inférieure de la plaque de support de patient inclinable, une barre transversale (430) s'étendant entre au moins deux montants de la pluralité de montants, et une équerre (400) couplée à la face inférieure de la plaque de support de patient inclinable au niveau d'une première extrémité de l'équerre, et couplée à la pluralité de montants, dans lequel l'équerre et la pluralité de montants forment un angle l'une avec l'autre, dans lequel l'équerre comprend une poignée (420) et de multiples encoches de retenue (425), et dans lequel l'équerre est configurée pour être réglée, via l'utilisation de la poignée, pour faire venir en prise sélectivement une encoche des multiples encoches de retenue avec la barre transversale pour régler l'angle d'inclinaison de la plaque de support de patient inclinable.

2. Système selon la revendication 1, dans lequel les multiples encoches de retenue sont disposées le long d'une face inférieure de l'équerre, et dans lequel la face inférieure de l'équerre se couple aux au moins deux montants de la pluralité de montants via la mise en prise de l'une des multiples encoches de retenue avec la barre transversale.

3. Système selon la revendication 1, comprenant en outre :
un pied tubulaire (415),
dans lequel la pluralité de montants se connecte en parallèle entre la face inférieure de la plaque de support de patient inclinable et le pied tubulaire et sont espacés les uns des autres.

4. Système selon la revendication 1, dans lequel les multiples encoches de retenue sont disposées, et espacées les unes des autres, le long d'une face inférieure de l'équerre et dans lequel une mise en prise sélective de chaque encoche différente des multiples encoches de retenue avec la barre transversale modifie l'angle d'inclinaison de la plaque de support de patient inclinable vers un angle différent parmi de multiples angles d'inclinaison.

5. Système selon la revendication 1, dans lequel l'équerre comprend une équerre de forme arquée, dans lequel la poignée comprend une poignée de réglage solidaire, et dans lequel les multiples encoches de retenue sont disposées le long d'une face inférieure de l'équerre pour permettre la modification d'un paramètre d'angle d'inclinaison du mécanisme de réglage d'angle pour régler l'angle d'inclinaison de la plaque de support de patient.

6. Système selon la revendication 1, comprenant en outre :
un pied (415) connecté transversalement à la pluralité de montants et configuré pour reposer sur une surface de support sous-jacente.

7. Système selon la revendication 6, comprenant en outre :
des pattes pivotantes (435) connectées à chaque extrémité du pied et configurées pour emprisonner des côtés de la surface de support sous-jacente.

8. Système selon la revendication 1, comprenant en outre :
une plaque de support inférieur de patient amovible qui se fixe de manière amovible à la plaque de support de patient inclinable au niveau d'un point de fixation articulé.

9. Système selon la revendication 8, dans lequel la plaque de support inférieur de patient se fixe de manière amovible à la plaque de support de patient inclinable via au moins un élément parmi des plongeurs à ressort libérables ou des boulons filetés.

10. Système selon la revendication 1, dans lequel la plaque de support de patient inclinable inclut une barre d'indexation qui vient en prise de manière coulissante avec une glissière à l'intérieur d'un évidement de la plaque de support de patient inclinable et dans lequel un module de support de partie de corps de patient se fixe de manière amovible à la barre d'indexation.

11. Système selon la revendication 1, dans lequel la plaque de support de patient inclinable comprend en outre :
un évidement disposé à l'intérieur d'une surface supérieure de la plaque de support de patient,
une glissière coulissante apposée à l'intérieur de l'évidement,
un cadre configuré pour coulisser sur la glissière coulissante, et
une barre d'indexation fixée au cadre et configurée pour permettre la fixation et le détachement d'un module de support de corps sur la barre d'indexation, et possédant en outre un plongeur à ressort libérable pour repositionner le module de support de corps au niveau d'une pluralité de positions le long de la glissière coulissante.

12. Système selon la revendication 1, dans lequel la plaque de support de patient inclinable supporte une partie d'un haut du corps d'un patient.

13. Système selon la revendication 1, comprenant en outre :
un module de support inférieur amovible qui se fixe à la plaque de support de patient inclinable au niveau de points de fixation articulés, dans lequel le module de support inférieur comprend en outre une plaque de support inférieur qui supporte une partie d'un bas du corps du patient et qui est rotatable, via les points de fixation articulés, pour amener la plaque de support inférieur à tourner vers une extrémité supérieure de la plaque de support de patient inclinable et à reposer sur une surface supérieure de la plaque de support de patient inclinable.

14. Système selon la revendication 13, dans lequel le module de support inférieur comprend en outre une butée inférieure surélevée qui se fixe de manière coulissante à la plaque de support inférieur.

15. Système selon la revendication 14, dans lequel le module de support inférieur comprend en outre un mécanisme à cliquet qui permet à la butée inférieure surélevée de coulisser sur la plaque de support inférieur vers une position parmi de multiples positions indexées sur la plaque de support inférieur.
